# EUROPÄISCHE PATENTSCHRIFT

(11) **EP 2 159 758 B1**
(45) Veröffentlichungstag und Bekanntmachung des Hinweises auf die Patenterteilung: **04.06.2014**
(21) Anmeldenummer: 09164983.0
(22) Anmeldetag: 09.07.2009
(51) Int. Cl.: G07C 5/08, G07C 3/08, B60T 10/02, F16D 57/00, G01N 33/28, F16H 57/04

(54) **Erfassen eines Verschleißzustandes eines Betriebsmediums und Festlegen eines Wechselintervalls**
Recording a wear status for an operating medium and setting a replacement interval
Détection d'un état d'usure d'un support de fonctionnement et fixation d'un intervalle de remplacement

(30) Priorität: 28.08.2008 DE 102008041639
(43) Veröffentlichungstag der Anmeldung: 03.03.2010
(73) Patentinhaber: ZF Friedrichshafen AG, 88038 Friedrichshafen (DE)
(72) Erfinder: Reisch, Bernhard, 88316 Isny (DE); Schmidtner, Peter, 88131 Lindau (DE)

(56) Entgegenhaltungen:
- DE-A1- 10 057 972
- DE-A1-102006 015 678
- US-A- 5 777 211
- US-A1- 2008 201 036

## Beschreibung

Die vorliegende Erfindung betrifft ein Verfahren zum Erfassen eines Verschleißzustandes eines Betriebsmediums bei einem Fahrzeuggetriebe, insbesondere mit einem Retarder, gemäß der im Oberbegriff des Patentanspruches 1 näher definierten Art. Darüber hinaus betrifft die Erfindung eine Anordnung zum Festlegen eines Wechselintervalls eines Betriebsmediums eines Fahrzeuggetriebes, insbesondere mit einem Retarder, gemäß der im Oberbegriff des Patentanspruches 7 näher definierten Art.

Beispielsweise aus der Druckschrift DE 100 57 972 A1 ist ein Verfahren zur Ermittlung von Bewertungskenngrößen für Getriebe in Fahrzeugen bekannt. Die erforderlichen Bewertungskenngrößen werden aus über Sensoren gemessenen Kenngrößen ermittelt. Als Kenngrößen können das Fahrzeuggewicht, der Fahrwiderstand, die Anzahl der Anfahrvorgänge des Fahrzeuges, die in einer Fahrzeugkupplung angefallene Reibarbeit, die maximal aufgetretene Öltemperatur oder die Betriebszeit des Getriebes verwendet werden. Die ermittelten Werte werden in einem Speicher der Getriebesteuerung abgelegt und können dem Fahrzeugführer oder dem Wartungspersonal über ein Display angezeigt werden. Mit den gewonnenen Bewertungskenngrößen kann der Werdegang des Getriebes dokumentiert werden und damit das Entstehen von Schäden im Getriebe aufgezeigt werden.

Ferner ist aus der Druckschrift DE 10 2006 015 678 A1 ein Verfahren zur Bestimmung des Zeitpunktes eines erforderlichen Wechsels für ein Betriebsmedium, insbesondere Öl, während des Betriebs eines Antriebsaggregates bekannt. Dazu wird für das im Antriebsaggregat verwendete Betriebsmittel eine Lebensdauerkennlinie vorgegeben. Die Temperatur des Betriebsmittels wird während des Betriebes des Antriebsaggregates überwacht und die Zeitdauer des Auftretens einer Temperatur bestimmter Größe als Einzelschaden zu einer Schadenssumme aufsummiert. Es wird eine Grenzschadenssumme vordefiniert, wobei die ermittelte Schadenssumme mit der Grenzschadenssumme verglichen wird. Bei Erreichen der Grenzschadenssumme wird für eine bestimmte Temperatur ein erforderlicher Wechsel des Betriebsmittels signalisiert. Ferner kann auch die verbleibende Restlebensdauer des Betriebsmittels berechnet und optisch angezeigt werden.

Die Ölwechselintervalle von Getrieben sind in der Regel fest vorgeschrieben und abhängig von der Laufleistung des Fahrzeugs. Wie bereits erwähnt nimmt die Alterung des Betriebsmittels bei höheren Temperaturen überproportional zu. Bei erschwerten Einsatzbedingungen, wie z.B. im Baustellenbetrieb, müssen die Wechselintervalle des Betriebsmittels verkürzt werden. Dagegen ist bei einer geringeren Belastung des Fahrzeuges eine längere Einsatzdauer des Betriebsmittels möglich. Aus Kosten- und Umweltschutzgründen ist es anzustreben, möglichst lange Wechselintervalle zu erhalten. Bei Getrieben mit Retarder-Systemen wird das Betriebsmedium bzw. das Getriebeöl im Bremsbetrieb zusätzlich erhitzt und damit die Alterung beschleunigt. Somit ist die Ölalterung im Getriebe stark abhängig vom Einsatzprofil des Fahrzeuges.

Der vorliegenden Erfindung liegt die Aufgabe zu Grunde, ein Verfahren zum Erfassen eines Verschleißzustandes eines Betriebsmediums eines Fahrzeuggetriebes und eine Anordnung zum Festlegen eines Wechselintervalls eines Betriebsmediums eines Fahrzeuggetriebes vorzuschlagen, bei denen der Wechselzeitpunkt des Betriebsmediums an einen vorbestimmten Zeitpunkt angepasst wird.

Diese Aufgabe wird erfindungsgemäß durch die Merkmale des Patentanspruches 1 beziehungsweise 7 gelöst. Weitere vorteilhafte Ausgestaltungen ergeben sich aus den Unteransprüchen.

Demnach wird ein Verfahren zum Erfassen eines Verschleißzustandes eines Betriebsmediums bei einem Fahrzeuggetriebe, insbesondere mit einem Retarder, vorgeschlagen, wobei die Temperatur des Betriebsmediums gemessen wird und jeweils ein aktueller Verschleißzustand des Betriebsmediums ermittelt wird, um die verbleibende Lebensdauer des Betriebsmediums zu bestimmen. Erfindungsgemäß wird der Betrieb des Fahrzeuggetriebes derart beeinflusst, dass ein geplanter Wechselzeitpunkt des Betriebsmediums mit dem Ende der Lebensdauer des Betriebsmediums zusammenfällt.

Auf diese Weise kann durch Beeinflussung der Betriebsweise des Fahrzeuggetriebes beziehungsweise des Retarders auch die Alterung beziehungsweise der Verschleißzustand beeinflusst werden, so dass demzufolge je nach Bedarf das Ende der Lebensdauer des Betriebsmediums variiert werden kann, um mit dem verwendeten Betriebsmedium noch den geplanten bzw. gewünschten Wechselzeitpunkt zu erreichen, ohne dass vor diesem Zeitpunkt das Ende der Lebensdauer des Betriebsmediums erreicht wird. Somit wird mit dem erfindungsgemäßen Verfahren eine optimale Ausnutzung des Betriebsmediums realisiert.

Um die aktuelle Alterung beziehungsweise den aktuellen Verschleißzustand des Betriebsmediums, wie z.B. des Getriebeöls, zu bestimmen, kann vorgesehen sein, dass die Getriebeöltemperatur an einer geeigneten Stelle zum Beispiel an dem Auslass des Retarders kontinuierlich gemessen wird. Mithilfe einer Kollektivierung der Öltemperaturen mit einem geeigneten Rechenalgorithmus kann dann der aktuelle Schädigungszustand des Betriebsmediums beziehungsweise des Getriebeöls ermittelt werden. Dazu kann auch eine Lebensdauerkennlinie des Betriebsmediums oder andere Parameter verwendet werden.

Gerade bei Getrieben mit Retardern ist die Alterung beziehungsweise der Verschleiß des Betriebsmediums beziehungsweise des Getriebeöls erheblich vom Einsatzprofil des Fahrzeuges abhängig. Insbesondere bei einem Einsatz bei hohen Umgebungstemperaturen und anspruchsvoller Topographie wird das Öl mehr geschädigt als bei einem Einsatz auf ebener Strecke bei normalen Umgebungstemperaturen. Wenn ein geplanter Wechselzeitpunkt des Getriebeöls bekannt ist, z.B. bei 500.000 gefahrenen Kilometern des Fahrzeuges, kann durch geeignete Algorithmen die Leistung des Fahrzeuggetriebes beziehungsweise des Retarders entsprechend angepasst bzw. begrenzt werden. Besonders vorteilhaft ist es, wenn das geplante Wechselintervall des Getriebeöls mit einem geplanten Wartungsintervall des Fahrzeuges zusammenfällt, bei dem z.B. das Motoröl gewechselt wird und andere Serviceleistungen durchgeführt werden. Mit dem erfindungsgemäßen Verfahren ist es somit möglich, dass sich das wählbare Getriebeölwechselintervall mit dem Wartungsintervall des Fahrzeuges deckt oder ein Vielfaches davon darstellt.

Gemäß einer möglichen Ausgestaltung der vorliegenden Erfindung kann vorgesehen sein, dass im Rahmen der Beeinflussung der Ansteuerung des Fahrzeuggetriebes die Bremsleistung des Retarders mithilfe geeigneter Algorithmen entsprechend variiert wird, so dass die geplante Solllaufzeit des Getriebeöls erreicht wird. Vorzugsweise kann die Bremsleistung des Retarders bei dem Fahrzeuggetriebe begrenzt werden, um die Temperatur des Getriebeöls zu verringern und die Lebensdauer bei Bedarf zu verlängern.

Wenn beispielsweise erkannt wird, dass die Schädigung des Getriebeöls geringer ist als bei der Auslegung angenommen, ist es auch möglich, dass die Bremsleistung des Retarders bei dem Fahrzeuggetriebe erhöht wird, um die Temperatur des Getriebeöls zu erhöhen und die Lebensdauer somit zu verkürzen.

Eine weitere vorteilhafte Ausführungsvariante der Erfindung kann vorsehen, dass der Wechselzeitpunkt des Getriebeöls manuell festgelegt wird und die Bremsleistungsansteuerung des Retarders entsprechend angepasst wird. Auf diese Weise kann zum Beispiel der Betreiber den Zeitpunkt des Ölwechsels selbst festlegen und die Bremsleistung des Retarders je nach dem entweder erhöhen oder verringern.

Die der Erfindung zu Grunde liegende Aufgabe wird auch durch eine Anordnung zum Festlegen eines Wechselintervalls eines Betriebsmediums eines Fahrzeuggetriebes, insbesondere mit einem Retarder, gelöst, wobei eine Eingabeeinrichtung zum Eingeben von vorbestimmten Betriebsparametern vorgesehen ist. Erfindungsgemäß kann als Eingabeeinrichtung ein Schalter oder dergleichen an einem Armaturenbrett des Fahrzeuges vorgesehen sein, um festzulegen, ob ein längeres Wechselintervall mit verminderter Bremsleistung des Retarders oder ein kürzeres Wechselintervall mit nicht verminderter Bremsleistung des Retarders verwendet wird. Somit ist ein benutzerseitiges Einstellen d es Wechselintervalls bzw. des Wechselzeitpunktes möglich.

Durch die vorliegende Erfindung kann eine Diagnose der Alterung beziehungsweise des Verschleißes eines Betriebsmediums, wie z.B. dem Getriebeöl, ermöglicht werden, um daraus eine entsprechende Ansteuerstrategie des Fahrzeuggetriebes, insbesondere des Retarders vorzusehen. Somit kann die verbleibende Lebensdauer an einen gewünschten Wechselzeitpunkt des Getriebeöls oder an einen geplanten Wartungszeitpunkt des Fahrzeuges angepasst werden.

Vorzugsweise kann die vorgeschlagene Anordnung mit dem erfindungsgemäßen Verfahren kombiniert werden, so dass die gewonnenen Informationen im Rahmen des Verfahrens von der Anordnung verarbeitet und ausgewertet werden, um die daraus resultierende Ansteuerstrategie des Fahrzeuggetriebes beziehungsweise des Retarders zu optimieren.

## Patentansprüche

1. Verfahren zum Erfassen eines Verschleißzustandes eines Betriebsmediums bei einem Fahrzeuggetriebe, insbesondere mit einem Retarder, wobei die Temperatur des Betriebsmediums gemessen wird und jeweils ein aktueller Verschleißzustand des Betriebsmediums ermittelt wird, um die verbleibende Lebensdauer des Betriebsmediums zu bestimmen, dadurch **gekennzeich net**, dass der Betrieb des Fahrzeuggetriebes derart beeinflusst wird, dass ein geplanter Wechselzeitpunkt des Betriebsmediums mit dem Ende der Lebensdauer des Betriebsmediums zusammenfällt.

2. Verfahren nach Anspruch 1, **dadurch gekennzeichnet, dass** das Wechselintervall des als Betriebsmedium vorgesehenen Getriebeöls mit einem geplanten Wartungsintervall des Fahrzeuges zusammenfällt.

3. Verfahren nach Anspruch 1 oder 2, **dadurch gekennzeichnet, dass** die Bremsleistung des Retarders bei dem Fahrzeuggetriebe begrenzt wird, um die Temperatur des Getriebeöls zu reduzieren und die Lebensdauer zu verlängern.

4. Verfahren nach einem der vorangehenden Ansprüche, dadurch **gek ennzeichnet**, dass die Bremsleistung des Retarders bei dem Fahrzeuggetriebe erhöht wird, um die Temperatur des Getriebeöls zu erhöhen und die Lebensdauer zu verkürzen.

5. Verfahren nach einem der vorangehenden Ansprüche, dadurch **gek ennzeichnet**, dass der Wechselzeitpunkt des Getriebeöls manuell festgelegt wird und die Bremsleistungsansteuerung des Retarders entsprechend angepasst wird.

6. Verfahren nach Anspruch 5, **dadurch gekennzeichnet, dass** manuell ausgewählt wird, ob ein langes Wechselintervall des Getriebeöls mit verminderter Bremsleistung des Retarders oder ein kürzeres Wechselintervall des Getriebeöls mit hoher Bremsleistung des Retarders verwendet wird.

7. Anordnung zum Festlegen eines Wechselintervalls eines Betriebsmediums eines Fahrzeuggetriebes, insbesondere mit einem Retarder, wobei eine Eingabeeinrichtung zum Eingeben von vorbestimmten Betriebsparametern vorgesehen ist, **dadurch gekennzeichnet, dass** als Eingabeeinrichtung ein Schalter an einem Armaturenbrett des Fahrzeuges vorgesehen ist, um festzulegen, ob ein längeres Wechselintervall mit verminderter Bremsleistung des Retarders oder ein kürzeres Wechselintervall ohne verminderte Bremsleistung des Retarders verwendbar ist.

## Claims

1. Method for sensing a wear status of an operating medium in a vehicle transmission, in particular having a retarder, wherein the temperature of the operating medium is measured and in each case a current wear status of the operating medium is detected in order to determine the remaining service life of the operating medium, **characterized in that** the operation of the vehicle transmission is influenced in such a way that a planned replacement time of the operating medium coincides with the end of the service life of the operating medium.

2. Method according to Claim 1, **characterized in that** the replacement interval of the gear oil which is provided as the operating medium coincides with a planned maintenance interval of the vehicle.

3. Method according to Claim 1 or 2, **characterized in that** the braking performance of the retarder is limited in the vehicle transmission in order to reduce the temperature of the gear oil and lengthen the service life.

4. Method according to one of the preceding claims, **characterized in that** the braking performance of the retarder is increased in the vehicle transmission in order to increase the temperature of the gear oil and shorten the service life.

5. Method according to one of the preceding claims, **characterized in that** the replacement time of the gear oil is defined manually, and the braking performance actuation of the retarder is correspondingly adapted.

6. Method according to Claim 5, **characterized in that** it is manually selected whether a long replacement interval of the gear oil with a reduced braking performance of the retarder or a relatively short replacement interval of the gear oil with a high braking performance of the retarder is used.

7. Arrangement for defining a replacement interval of an operating medium of a vehicle transmission, in particular having a retarder, wherein an input device for inputting predetermined operating parameters is provided, **characterized in that** a switch is provided as an input device on a dashboard of the vehicle in order to define whether a relatively long replacement interval with a reduced braking performance of the retarder or a relatively short replacement interval without reduced braking performance of the retarder can be used.

## Revendications

1. Procédé de détection d'un état d'usure d'un milieu de travail dans une transmission de véhicule, en particulier avec un ralentisseur, la température du milieu de travail étant mesurée et un état d'usure actuel du milieu de travail étant à chaque fois déterminé afin de déterminer la durée de vie restante du milieu de travail, **caractérisé en ce que** le fonctionnement de la transmission du véhicule est influencé de telle sorte qu'un instant de remplacement prévu du milieu de travail coïncide avec la fin de la durée de vie du milieu de travail.

2. Procédé selon la revendication 1, **caractérisé en ce que** l'intervalle de remplacement de l'huile de transmission prévue en tant que milieu de travail coïncide avec un intervalle d'entretien prévu du véhicule.

3. Procédé selon la revendication 1 ou 2, **caractérisé en ce que** la puissance de freinage du ralentisseur dans la transmission du véhicule est limitée afin de réduire la température de l'huile de transmission et de prolonger la durée de vie.

4. Procédé selon l'une quelconque des revendications précédentes, **caractérisé en ce que** la puissance de freinage du ralentisseur dans la transmission du véhicule est augmentée afin d'augmenter la température de l'huile de transmission et de raccourcir la durée de vie.

5. Procédé selon l'une quelconque des revendications précédentes, **caractérisé en ce que** l'instant de remplacement de l'huile de transmission est fixé manuellement et la commande de puissance de freinage du ralentisseur est adaptée en conséquence.

6. Procédé selon la revendication 5, **caractérisé en ce que** l'on sélecte manuellement si l'on utilise un intervalle de remplacement long de l'huile de transmission avec une puissance de freinage réduite du ralentisseur ou un intervalle de remplacement plus court de l'huile de transmission avec une grande puissance de freinage du ralentisseur.

7. Agencement pour fixer un intervalle de remplacement d'un milieu de travail d'une transmission de véhicule, en particulier comprenant un ralentisseur, un dispositif d'entrée pour entrer des paramètres de fonctionnement prédéterminés étant prévu, **caractérisé en ce que** l'on prévoit en tant que dispositif d'entrée un commutateur sur un tableau de bord du véhicule, afin d'établir si l'on peut utiliser un intervalle de remplacement plus long avec une puissance de freinage réduite du ralentisseur ou un intervalle de remplacement plus court sans puissance de freinage réduite du ralentisseur.
